# EUROPEAN PATENT SPECIFICATION

(11) **EP 3 997 714 B1**
(45) Date of publication and mention of the grant of the patent: **28.08.2024**
(21) Application number: 20735676.7
(22) Date of filing: 26.06.2020
(51) Int. Cl.: G16H 50/70, G16B 15/30, G16B 40/30, G16C 20/40, G16C 20/50

(54) **IDENTIFYING ONE OR MORE COMPOUNDS FOR TARGETING A GENE**
IDENTIFIZIERUNG VON EINER ODER MEHREREN VERBINDUNGEN ZUM TARGETING EINES GENS
IDENTIFICATION D'UN OU DE PLUSIEURS COMPOSÉ(S) POUR LE CIBLAGE D'UN GÈNE

(30) Priority: 10.07.2019 GB 201909925
(43) Date of publication of application: 18.05.2022
(73) Proprietor: BenevolentAI Technology Limited, London Greater London W1T 5HD (GB)
(72) Inventor: SELLWOOD, Matthew, London Greater London E17 4SR (GB)
(74) Representative: Gill Jennings & Every LLP
(86) International application number: PCT/GB2020/051549
(87) International publication number: WO 2021/005332

(56) References cited:
- US-A1- 2019 010 533
- US-A1- 2019 114 390
- DUAN Q ET AL: "L1000CDS2: LINCS L1000 characteristic direction signatures search engine", NPJ SYSTEMS BIOLOGY AND APPLICATIONS, vol. 2, no. 1, 4 August 2016 (2016-08-04), XP093078769, Retrieved from the Internet <URL:https://www.nature.com/articles/npjsba201615.pdf> DOI: 10.1038/npjsba.2016.15
- LAGUNIN A ET AL: "DIGEP-Pred: web service for in silico prediction of drug-induced gene expression profiles based on structural formula", BIOINFORMATICS, vol. 29, no. 16, 5 June 2013 (2013-06-05), GB, pages 2062 - 2063, XP093078765, ISSN: 1367-4803, Retrieved from the Internet <URL:https://academic.oup.com/bioinformatics/article-pdf/29/16/2062/49077065/bioinformatics_29_16_2062.pdf> DOI: 10.1093/bioinformatics/btt322
- IORIO F ET AL: "Discovery of drug mode of action and drug repositioning from transcriptional responses", PROCEEDINGS OF THE NATIONAL ACADEMY OF SCIENCES, vol. 107, no. 33, 17 August 2010 (2010-08-17), pages 14621 - 14626, XP055057022, ISSN: 0027-8424, DOI: 10.1073/pnas.1000138107

## Description

The present application relates to systems and methods for identifying tool compounds. Tool compounds are compounds that can be used to target a gene in order to test whether the gene is associated with a disease under study.

### Background

In the field of drug discovery, a disease-target hypothesis is a hypothesis that a disease is associated with a target gene. In order to test a disease-target hypothesis, drug discovery scientists are interested in knowing which are the best tool compounds that can be used to target the gene.

However, the process of identifying the most effective and commercially viable tool compound for testing a gene is time-intensive, and this introduces significant delays and costs into the program of drug discovery.

A technique for more efficiently identifying tool compounds for target genes is needed to help enable rapid, high-volume validation of disease-target hypotheses.

The embodiments described below are not limited to implementations which solve any or all of the disadvantages of the known approaches described above.

US 2019/0010533 describes methods for screening and selecting target agents from molecular databases. This document does not disclose predicting genes associated with the first candidate compound using a machine learning model trained to predict a gene interaction profile for a range of compounds, or building fingerprints and carrying out filtering operations based thereon.

### Summary

This Summary is provided to introduce a selection of concepts in a simplified form that are further described below in the Detailed Description. This Summary is not intended to identify key features or essential features of the claimed subject matter, nor is it intended to be used to determine the scope of the claimed subject matter.

In a first aspect, the present disclosure provides a computer-implemented method of identifying a compound for targeting one or more target genes, as defined in the appended claims.

The method comprises searching a database for first candidate compounds that each target one or more first target genes; generating a first fingerprint for each first candidate compound by: searching the database for genes associated with the first candidate compound, predicting genes associated with the first candidate compound using a machine learning model trained to predict a gene interaction profile for a range of compounds, and building the first fingerprint using the genes returned by the search and the predicted genes; and filtering the first candidate compounds using the first fingerprints to identify a first optimum compound for targeting the one or more first target genes, wherein filtering the first candidate compounds comprises comparing each of the first fingerprints to an ideal fingerprint of a theoretical tool compound, where each fingerprint comprises a polypharmacology fingerprint that describes which genes the compound is associated with.

Predicting genes associated with the first candidate compound comprises using a machine learning model trained to predict a gene interaction profile with a range of compounds.

Optionally, the model may comprise a neural network.

Optionally, the method may comprise predicting genes associated with the first candidate compound only when there is no association data available in the database.

Filtering the first candidate compounds comprises comparing each of the first fingerprints to an ideal fingerprint of a theoretical tool compound.

Optionally, the comparing may comprise calculating a similarity score.

Optionally, the method comprises identifying a first candidate compound that is most similar to the theoretical tool compound as the first optimum compound.

Filtering the first candidate compounds comprises generating metrics using the first fingerprints and filtering the first candidate compounds using the metrics.

Optionally, generating the first fingerprints may comprise obtaining metadata about one or more of the first candidate compounds.

Optionally, the metadata may comprise clinical trial phase data, a drug name or property, or information from a compound vendor.

Optionally, the method may comprise using a library evaluation framework to retrieve an indication of how many targets each first candidate compound has.

Optionally, the method may comprise: searching the database for second candidate compounds that each target one or more second target genes; generating a second fingerprint for each second candidate compound by: searching the database for genes associated with the second candidate compound, and predicting genes associated with the second candidate compound; and filtering a group comprising the first candidate compounds and the second candidate compounds using the first fingerprints and the second fingerprints to identify the first optimum compound and to identify a second optimum compound for targeting the one or more second target genes.

In a second aspect, the present disclosure provides a system for identifying a compound for targeting one or more target genes, as defined in the appended claims. The system comprises: a compound search module configured to search a database for first candidate compounds that each target one or more first target genes; a fingerprint module configured to generate a first fingerprint for each first candidate compound, the fingerprint module comprising: a gene search module configured to search the database for genes associated with the first candidate compound, and a prediction module configured to predict genes associated with the first candidate compound using a machine learning model trained to predict a gene interaction profile for a range of compounds, fingerprint module configured to build the first fingerprint using the genes returned by the gene search module and the prediction module; and a filter module configured to filter the first candidate compounds using the first fingerprints to identify a first optimum compound for targeting the one or more first target genes, wherein filtering the first candidate compounds comprises comparing each of the first fingerprints to an ideal fingerprint of a theoretical tool compound, where each fingerprint comprises a polypharmacology fingerprint that describes which genes the compound is associated with.

The prediction module is configured to use a model trained to predict a gene interaction profile with a range of compounds.

Optionally, the model may comprise a neural network.

Optionally, the prediction module may be configured to predict genes associated with the first candidate compound only when there is no association data available in the database.

The filter module is configured to filter the first candidate compounds by comparing each of the first fingerprints to an ideal fingerprint of a theoretical tool compound.

Optionally, the comparing may comprise calculating a similarity score.

Optionally, the filter module may be configured to identify one or more of the first candidate compounds which are most similar to the ideal tool compound.

Optionally, the filter module may be configured to select, as the first optimum compound, the first candidate compound that is the most similar to the ideal tool compound.

Optionally, the fingerprint module may be configured to obtain metadata about one or more of the first candidate compounds.

Optionally, the metadata may comprise clinical trial phase data, a drug name or property, or information from a compound vendor.

Optionally, the fingerprint module may be configured to use a library evaluation framework to retrieve an indication of how many targets each first candidate compound has.

Optionally, the compound search module may be configured to search the database for second candidate compounds that each target one or more second target genes; the fingerprint module may be configured to generate a second fingerprint for each second candidate compound; the gene search module may be configured to search the database for genes associated with the second candidate compound; the prediction module may be configured to predict genes associated with the second candidate compound; and the filter module may be configured to filter a group comprising the first candidate compounds and the second candidate compounds using the first fingerprints and the second fingerprints to identify the first optimum compound and to identify a second optimum compound for targeting the one or more second target genes.

In a third aspect, the present disclosure provides a computer-readable medium storing code that, when executed by a computer, causes the computer to perform the method of the first aspect.

The methods described herein may be performed by software in machine readable form on a tangible storage medium e.g. in the form of a computer program comprising computer program code means adapted to perform all the steps of any of the methods described herein when the program is run on a computer and where the computer program may be embodied on a computer readable medium. Examples of tangible (or non-transitory) storage media include disks, thumb drives, memory cards etc. and do not include propagated signals. The software can be suitable for execution on a parallel processor or a serial processor such that the method steps may be carried out in any suitable order, or simultaneously.

This application acknowledges that firmware and software can be valuable, separately tradable commodities. It is intended to encompass software, which runs on or controls "dumb" or standard hardware, to carry out the desired functions. It is also intended to encompass software which "describes" or defines the configuration of hardware, such as HDL (hardware description language) software, as is used for designing silicon chips, or for configuring universal programmable chips, to carry out desired functions.

The preferred features may be combined as appropriate, as would be apparent to a skilled person, and may be combined with any of the aspects of the invention.

### Brief Description of the Drawings

Embodiments of the invention will be described, by way of example, with reference to the following drawings, in which:
Figure 1 is a schematic diagram representing an embodiment of the invention for identifying an optimum tool compound for targeting a target gene;
Figure 2 is a flow chart of a method of identifying an optimum tool compound for targeting a target gene;
Figure 3 is a schematic diagram of a polypharmacology fingerprint of a candidate compound;
Figure 4 is a schematic diagram representing an embodiment of the invention for identifying an optimum tool compound for targeting a gene set;
Figure 5 is a schematic diagram representing an embodiment of the invention for identifying respective optimum tool compounds for targeting respective target genes;
Figure 6 is a is a schematic diagram representing an embodiment of the invention for identifying respective optimum tool compounds for targeting respective gene sets;
Figure 7 is a block diagram of a system according to an embodiment of the invention; and
Figure 8 is a block diagram of a computer suitable for implementing embodiments of the invention.

Common reference numerals are used throughout the figures to indicate similar features.

### Detailed Description

Embodiments of the present invention are described below by way of example only. These examples represent the best ways of putting the invention into practice that are currently known to the Applicant although they are not the only ways in which this could be achieved. The description sets forth the functions of the example and the sequence of steps for constructing and operating the example. However, the same or equivalent functions and sequences may be accomplished by different examples.

The present invention provides an automated way of generating candidate compounds for targeting a gene and filtering the candidate compounds to identify an optimum tool compound or a shortlist of optimum tool compounds. This enables a drug discovery scientist to rapidly identify one or more optimum tool compounds for targeting a gene in order to test a disease-target hypothesis.

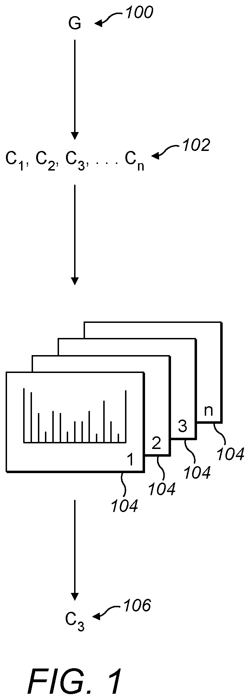

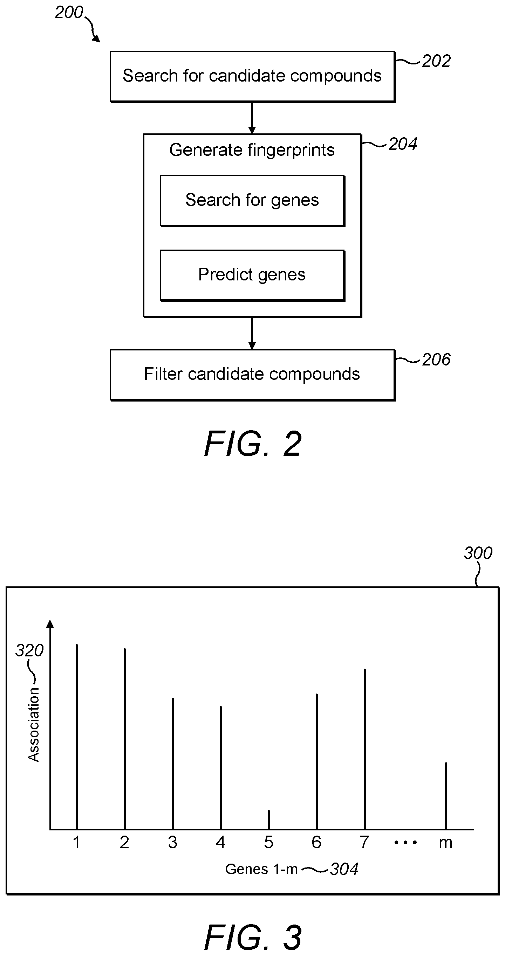

Referring to Figures 1 and 2, a drug discovery scientist may want to identify a tool compound for targeting a gene G 100. As shown in Figure 2, a method 200 of identifying a tool compound in accordance with an embodiment of the invention comprises searching 202 a database for candidate compounds 102 that target the gene G 100. As shown in Figure 1, this search results in *n* candidate compounds C₁-Cₙ 102 which may be suitable for targeting the gene G 100. In this disclosure, the term 'database' is used to refer to one or more databases. Each of the one or more databases may comprise a distributed database. Searching the one or more databases may comprise using an application program interface (API) to conduct the search.

The database may comprise a compound database that can be searched to find compounds that are associated with the gene G. In suitable examples, the compounds database may store structured data from a range of public sources, including but not limited to chemical databases, patents, and predictions between pairs of compounds and target genes. The database may additionally or alternatively comprise unstructured data such as patents or articles, and may also include processed unstructured data. As such, associations extracted from the database have generally been verified experimentally. Any compounds that are identified in the search as being associated with the gene G are then candidate compounds for targeting the gene G.

A stage of analysis then follows in which the candidate compounds C₁-Cₙ 102 are characterised and filtered in order to identify one or more tool compounds with optimum characteristics for targeting the gene G 100. In order to characterise the candidate compounds C₁-Cₙ 102, a fingerprint 104 is generated 204 for each that describes the characteristics and properties of the respective candidate compound in such a way as to enable the candidate compounds C₁-Cₙ 102 to be assessed.

A useful factor for determining the suitability of the candidate compounds C₁-Cₙ 102 is which genes they are associated with. As a result, the fingerprint 104 of each candidate compound 102 comprises a polypharmacology fingerprint that describes which genes the candidate compound 102 is associated with. For example, a polypharmacology fingerprint 300 for a candidate compound 102 is shown in Figure 3. For each of a range of genes G₁-Gₘ 304, the polypharmacology fingerprint 300 comprises a representation of whether each respective gene 304 is associated with the candidate compound 102. In the example of Figure 3, a representation of an extent of association 302 is provided which may, for example, represent an extent of upregulation of each respective gene 304 by the candidate compound 102. In other examples, a polypharmacology fingerprint may also show genes that are inhibited by the candidate compound 102 and an extent to which they are inhibited. In any case, a polypharmacology fingerprint describes the activity of a compound with respect to a preferably large number of genes, and can be used to filter the candidate compounds 102 to find a single optimum compound, or multiple optimal compounds, for targeting the gene G.

In order to build a polypharmacology fingerprint for a candidate compound 102, data is required relating to the candidate compound 102 and a range of genes. Genes that are associated with the candidate compound 102, for example by upregulating or inhibiting it, are identified in two ways.

Firstly, the above-mentioned database is searched for genes associated with the candidate compound 102. Data relating to the nature the associations, such as whether they are upregulations or inhibitions and by how much, may be retrieved in this search.

Furthermore, metadata about compounds from vendors may be extracted in this search. Examples of metadata from vendors include live availability of stock and price information. Metadata from other vendors or other sources may include the phase of clinical trial a molecule has been in, and the name of a molecule if it is a drug (for example, celecoxib). Additionally or alternatively, a suitable tool such as a library evaluation framework may be used to retrieve information relating to how many targets are identified in relation to a set of candidate compounds. Such a tool provides a quick, easy and interpretable way of quantitatively assessing the library before purchasing it or using it in biological experiments. This is advantageous as there is often limited information available on the quality of the molecules provided as part of the library when it is purchased from a vendor.

Secondly, to make the polypharmacology fingerprint more extensive or if there is no association data available for the candidate compound 102, a model is used to predict which genes have associations with the candidate compound 102. Association data comprises experimental data, for example from a biological assay, that is reported in the literature and retrievable from a database. The association data indicates an association between the candidate compound 102 and a gene, and may for example comprise binding data of the candidate compound 102 to a target gene, or alternatively may comprise drug metabolism and pharmacokinetics (DMPK) and/or absorption, distribution, metabolism, and excretion (ADMET) properties of the candidate compound 102 such as solubility, metabolic stability, and so on.

Associations may be predicated using a trained machine learning algorithm such as a neural network or any other suitable machine learning model. The choice of machine learning model may be influenced by the size of the dataset available for training. For example, for large datasets a random forest algorithm may be suitable, while for small datasets a transfer learning algorithm may be preferred.

Any data source that describes interactions between genes and compounds may be used. In suitable examples, the machine learning model predicts an association between a compound and a gene based on a known association between the same compound and a similar or related gene, for example a gene wit ha similar binding site. In other suitable examples, the machine learning model predicts interactions between compounds and gene binding sites using three-dimensional interaction data. **]**Three-dimensional interaction data may comprise data relating to the conformation of the molecule or compound in three spatial dimensions or may comprise data relating to the structure of at least part of a gene in three spatial dimensions. By virtue of the predictions, the machine learning model determines which compounds and genes are associated with each other.

This process is repeated to generate a fingerprint for each of the candidate compounds C₁-Cₙ 102.

Once a full set of fingerprints has been generated for the candidate compounds C₁-Cₙ 102, the candidate compounds C₁-Cₙ 102 are filtered 206 using the fingerprints to obtain either a list of optimum tool compounds or a single optimum tool compound 106 for targeting the gene G 100.

There are various ways of filtering the candidate compounds 102. The fingerprints can be compared to an ideal fingerprint of a theoretical tool compound to identify fingerprints that are most similar to the ideal fingerprint. This comparison may comprise calculating a similarity score between each fingerprint and the ideal fingerprint of the theoretical tool compound. The candidate compound having the highest similarity score is selected as the optimum tool compound, or alternatively, if multiple tool compounds are required, the candidate compounds having the highest similarity scores are selected as tool compounds.

According to the invention, metrics are generated from the fingerprints and used to filter the candidate compounds. For example, metrics may include but are not limited to default scoring metrics such as those related to physical or chemical properties. The metrics comprise one or more of: molar weight (MW), the logarithm of the partition coefficient (logP), the number of hydrogen bond acceptors or donors and so on, or enzyme activity such as values of the half maximal inhibitory concentration (IC50) of the molecule or the half maximal effective concentration of the molecule (EC50) in assay, selectivity of a compound for a target gene, number of off-targets (i.e. other unwanted genes that the compound affects), potency of the compound for a gene, solubility, cell data providing an indication of the activity of a compound in a cellular assay, and commercial availability. The metrics used may be user-selected and additionally or alternatively may be weighted by importance by the user. A combination of the metrics may be used to generate an aggregate score for each candidate compound.

The invention uses a combination of filtering the candidate compounds by comparing the fingerprints to an ideal fingerprint and filtering the candidate compounds by generating metrics from the fingerprints.

The present invention can be used to identify tool compounds that are distinct from each other. If two compounds are identified that target the same gene but have different off-targets, this can be used to increase the confidence that the target gene is relevant to the treatment mechanism of a disease if both compounds have a beneficial effect in treating the disease.

In the above embodiment, the invention is used to find one or more optimum tool compounds for targeting a single gene. However, there are some situations in which a drug discovery scientist may wish to find a single compound that targets multiple genes, for example for the effective treatment of a disease with a more complicated disease mechanism. In this situation an alternative embodiment may be used to find one or more optimum compounds for targeting a set of genes.

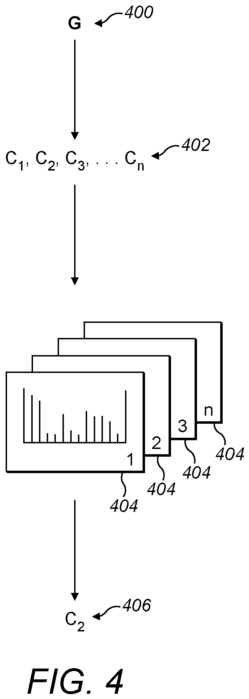

Referring to Figure 4, a gene set **G** 400 comprising a plurality of genes is used to search a database for compounds that are associated with one or more of the genes of the gene set **G** 400. Compounds 402 that are returned in the search are candidate compounds 402 for targeting genes of the gene set **G** 400, and may simultaneously target all the members in the gene set **G** 400. A fingerprint 404 is generated for each candidate compound 402 and used to filter the candidate compounds 402. The ideal fingerprint for a theoretical compound in this case will be that which describes the ideal interactions of a tool compound with all the genes in the gene set **G** 400. This enables the identification of one or more optimum tool compounds 406 for targeting the genes of the gene set **G** 400.

There may be situations in which a drug discovery scientist wishes to use the above embodiment of Figure 1 more than once to identify respective optimum tool compounds for respective target genes. For example, the drug discovery scientist may wish to identify a first optimum tool compound for targeting a first target gene and a second optimum tool compound for targeting a second target gene. In this case, the embodiment of Figure 1 may be run in parallel to identify the respective optimum tool compounds simultaneously.

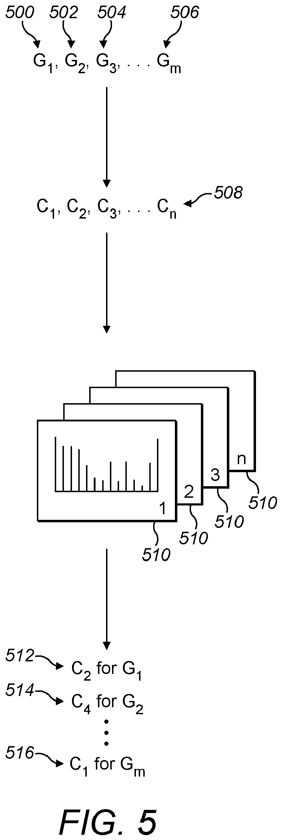

In an example of this approach, a respective tool compound is needed for targeting each of a plurality of genes G₁ 500, G₂ 502, G₃ 504 and Gₘ 506. Referring to Figure 5, a database is searched to identify compounds that have an association with one or more of the genes G₁ 500, G₂ 502, G₃ 504 and Gₘ 506. The compounds 508 that are identified in the search are candidate compounds 508 for targeting the respective genes. A fingerprint 510 is generated for each candidate compound 508 and used to filter the candidate compounds 508. This enables the identification of a respective optimum tool compound 512, 514, 516 for each of the genes G₁ 500, G₂ 502, G₃ 504 and Gₘ 506. If multiple tool compounds are required for each gene, this approach may also be used to identify a respective plurality of optimum tool compounds for each of the genes G₁ 500, G₂ 502, G₃ 504 and Gₘ 506.

Similarly, there may be situations in which a drug discovery scientist wishes to use the above embodiment of Figure 4 more than once to identify respective optimum tool compounds for targeting respective gene sets. For example, the drug discovery scientist may wish to identify a first optimum tool compound for targeting a first gene set and a second optimum tool compound for targeting a second gene set. In this case, the embodiment of Figure 4 may be run in parallel to identify the respective optimum tool compounds simultaneously.

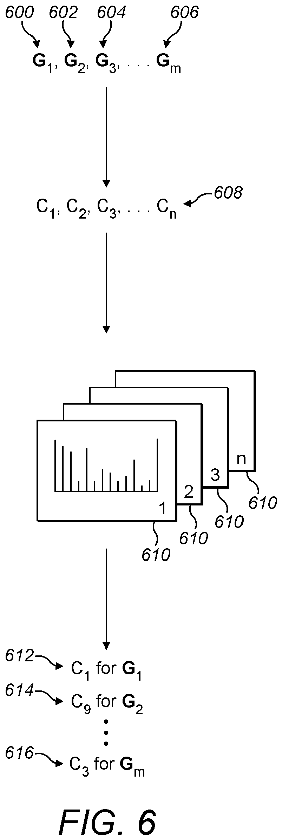

In an example of this approach, a respective tool compound is needed for targeting each of a plurality of gene sets **G₁** 600, **G₂** 602, **G₃** 604 and **Gₘ** 606. Referring to Figure 6, a database is searched to identify compounds that have an association with one or more of the gene sets **G₁** 600, **G₂** 602, **G₃** 604 and **G**ₘ 606. The compounds 608 that are identified in the search are candidate compounds 608 for targeting the respective gene sets **G₁** 600, **G₂** 602, **G₃** 604 and **Gₘ** 606. A fingerprint 610 is generated for each candidate compound 608 and used to filter the candidate compounds 608. This enables the identification of a respective optimum tool compound 612, 614, 616 for each of the gene sets **G₁** 600, **G₂** 602, **G₃** 604 and **Gₘ** 606. If multiple tool compounds are required for each gene set, this approach may also be used to identify a respective plurality of optimum tool compounds for each of the gene sets **G₁** 600, **G**₂ 602, **G₃** 604 and **Gₘ** 606.

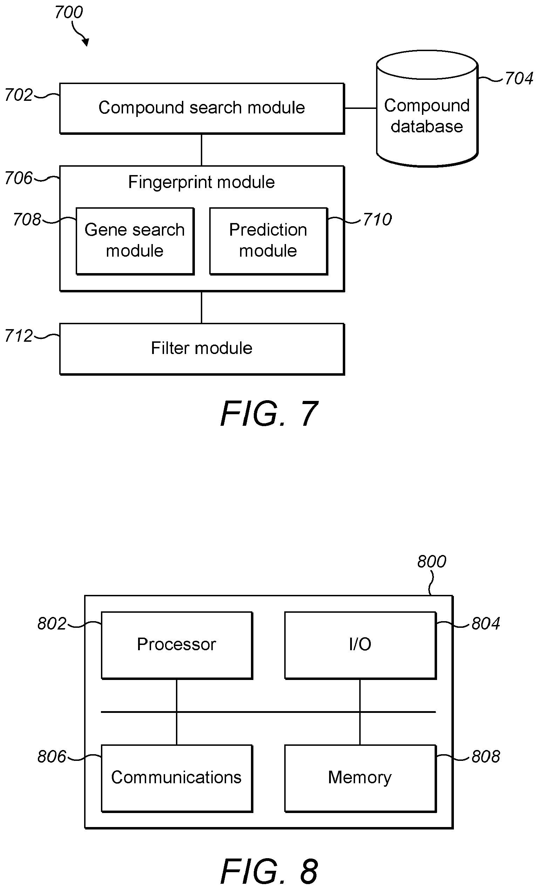

A system 700 for identifying a tool compound according to the present invention is shown in Figure 7. The system comprises a compound search module 702 configured to search a database 704 for candidate compounds that each target one or more target genes. The system 700 also comprises a fingerprint module 706 configured to generate a fingerprint for each candidate compound . The fingerprint module 706 comprises a gene search module 708 configured search the database 704 for genes associated with each candidate compound and a prediction module 710 configured to predict genes associated with each candidate compound. Finally, the system 700 also comprises a filter module 712 configured to filter the candidate compounds using the fingerprints to identify an optimum compound for targeting the one or more target genes.

A computer apparatus 800 suitable for implementing methods according to the present invention is shown in Figure 8. The apparatus 800 comprises a processor 802, an input-output device 804, a communications portal 806 and computer memory 808. For example, the memory 808 may store code that, when executed by the processor 802, causes the apparatus 800 to perform the method 200 shown in Figure 2.

In the embodiment described above the server may comprise a single server or network of servers. In some examples the functionality of the server may be provided by a network of servers distributed across a geographical area, such as a worldwide distributed network of servers, and a user may be connected to an appropriate one of the network of servers based upon a user location.

The above description discusses embodiments of the invention with reference to a single user for clarity. It will be understood that in practice the system may be shared by a plurality of users, and possibly by a very large number of users simultaneously.

The embodiments described above are fully automatic. In some examples a user or operator of the system may manually instruct some steps of the method to be carried out.

In the described embodiments of the invention the system may be implemented as any form of a computing and/or electronic device. Such a device may comprise one or more processors which may be microprocessors, controllers or any other suitable type of processors for processing computer executable instructions to control the operation of the device in order to gather and record routing information. In some examples, for example where a system on a chip architecture is used, the processors may include one or more fixed function blocks (also referred to as accelerators) which implement a part of the method in hardware (rather than software or firmware). Platform software comprising an operating system or any other suitable platform software may be provided at the computing-based device to enable application software to be executed on the device.

Various functions described herein can be implemented in hardware, software, or any combination thereof. If implemented in software, the functions can be stored on or transmitted over as one or more instructions or code on a computer-readable medium. Computer-readable media may include, for example, computer-readable storage media. Computer-readable storage media may include volatile or non-volatile, removable or non-removable media implemented in any method or technology for storage of information such as computer readable instructions, data structures, program modules or other data. A computer-readable storage media can be any available storage media that may be accessed by a computer. By way of example, and not limitation, such computer-readable storage media may comprise RAM, ROM, EEPROM, flash memory or other memory devices, CD-ROM or other optical disc storage, magnetic disc storage or other magnetic storage devices, or any other medium that can be used to carry or store desired program code in the form of instructions or data structures and that can be accessed by a computer. Disc and disk, as used herein, include compact disc (CD), laser disc, optical disc, digital versatile disc (DVD), floppy disk, and blu-ray disc (BD). Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable,twisted pair, DSL, or wireless technologies such as infrared, radio, and microwave are included in the definition of communication medium. Combinations of the above should also be included within the scope of computer-readable media.

Alternatively, or in addition, the functionality described herein can be performed, at least in part, by one or more hardware logic components. For example, and without limitation, hardware logic components that can be used may include Field-programmable Gate Arrays (FPGAs), Program-specific Integrated Circuits (ASICs), Program-specific Standard Products (ASSPs), System-on-a-chip systems (SOCs). Complex Progrmmable Logic Devices (CPLDs), etc.

Although illustrated as a single system, it is to be understood that the computing device may be a distributed system. Thus, for instance, several devices may be in communication by way of a network connection and may collectively perform tasks described as being performed by the computing device.

Although illustrated as a local device it will be appreciated that the computing device may be located remotely and accessed via a network or other communication link (for example using a communication interface).

The term 'computer' is used herein to refer to any device with processing capability such that it can execute instructions. Those skilled in the art will realise that such processing capabilities are incorporated into many different devices and therefore the term 'computer' includes PCs, servers, mobile telephones, personal digital assistants and many other devices.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed, or execute some software instructions at the local terminal and some at the remote computer (or computer network). Those skilled in the art will also realise that by utilising conventional techniques known to those skilled in the art that all, or a portion of the software instructions may be carried out by a dedicated circuit, such as a DSP, programmable logic array, or the like.

It will be understood that the benefits and advantages described above may relate to one embodiment or may relate to several embodiments. The embodiments are not limited to those that solve any or all of the stated problems or those that have any or all of the stated benefits and advantages.

Any reference to 'an' item refers to one or more of those items. The term 'comprising' is used herein to mean including the method steps or elements identified, but that such steps or elements do not comprise an exclusive list and a method or apparatus may contain additional steps or elements.

As used herein, the terms "component" and "system" are intended to encompass computer-readable data storage that is configured with computer-executable instructions that cause certain functionality to be performed when executed by a processor. The computer-executable instructions may include a routine, a function, or the like. It is also to be understood that a component or system may be localized on a single device or distributed across several devices.

Further, as used herein, the term "exemplary" is intended to mean "serving as an illustration or example of something".

Further, to the extent that the term "includes" is used in either the detailed description or the claims, such term is intended to be inclusive in a manner similar to the term "comprising" as "comprising" is interpreted when employed as a transitional word in a claim.

The figures illustrate exemplary methods. While the methods are shown and described as being a series of acts that are performed in a particular sequence, it is to be understood and appreciated that the methods are not limited by the order of the sequence. For example, some acts can occur in a different order than what is described herein. In addition, an act can occur concurrently with another act. Further, in some instances, not all acts may be required to implement a method described herein.

Moreover, the acts described herein may comprise computer-executable instructions that can be implemented by one or more processors and/or stored on a computer-readable medium or media. The computer-executable instructions can include routines, sub-routines, programs, threads of execution, and/or the like. Still further, results of acts of the methods can be stored in a computer-readable medium, displayed on a display device, and/or the like.

The order of the steps of the methods described herein is exemplary, but the steps may be carried out in any suitable order, or simultaneously where appropriate. Additionally, steps may be added or substituted in, or individual steps may be deleted from any of the methods without departing from the scope of the subject matter described herein. Aspects of any of the examples described above may be combined with aspects of any of the other examples described to form further examples without losing the effect sought.

It will be understood that the above description of a preferred embodiment is given by way of example only and that various modifications may be made by those skilled in the art. What has been described above includes examples of one or more embodiments. It is, of course, not possible to describe every conceivable modification and alteration of the above devices or methods for purposes of describing the aforementioned aspects, but one of ordinary skill in the art can recognize that many further modifications and permutations of various aspects are possible. Accordingly, the described aspects are intended to embrace all such alterations, modifications, and variations that fall within the scope of the appended claims.

## Claims

1. A computer-implemented method of identifying a compound for targeting one or more target genes, the method comprising:
searching a database for first candidate compounds that each target one or more first target genes;
generating a first fingerprint for each first candidate compound by:
searching the database for genes associated with the first candidate compound,
predicting genes associated with the first candidate compound using a machine learning model trained to predict a gene interaction profile for a range of compounds, and
building the first fingerprint using the genes returned by the search and the predicted genes; and
filtering the first candidate compounds using the first fingerprints to identify a first optimum compound for targeting the one or more first target genes, wherein filtering the first candidate compounds comprises:
combining filtering by comparing each of the first fingerprints to an ideal fingerprint of a theoretical tool compound and filtering by using metrics generated from the first fingerprints,
wherein the metrics comprise one or more of: molar weight (MW), the logarithm of the partition coefficient (logP), the number of hydrogen bond acceptors or donors, enzyme activity such as values of the half maximal inhibitory concentration (IC50) of the compound or the half maximal effective concentration of the compound (EC50) in assay, selectivity of a compound for a target gene, number of unwanted genes that the compound affects, potency of the compound for a gene, solubility, and cell data providing an indication of the activity of a compound in a cellular assay,
where each fingerprint comprises a polypharmacology fingerprint that describes which genes the compound is associated with.

2. The computer-implemented method of claim 1, wherein each fingerprint for a compound represents the extent of upregulation of each of a number of genes associated with the compound.

3. The computer-implemented method of any preceding claim, wherein each fingerprint for a compound represents the extent to which each of a number of genes are inhibited by the compound.

4. The computer-implemented method of any preceding claim, wherein the model comprises a neural network.

5. The computer-implemented method of any preceding claim, wherein the comparing comprises calculating a similarity score.

6. The computer-implemented method of any preceding claim, comprising identifying a first candidate compound that is most similar to the theoretical tool compound as the first optimum compound.

7. The computer-implemented method of any preceding claim, wherein generating the first fingerprints comprises obtaining metadata about one or more of the first candidate compounds, wherein the metadata comprises clinical trial phase data, a drug name or property, or information from a compound vendor.

8. The computer-implemented method of any preceding claim, comprising:
searching the database for second candidate compounds that each target one or more second target genes;
generating a second fingerprint for each second candidate compound by:
searching the database for genes associated with the second candidate compound, and
predicting genes associated with the second candidate compound using a machine learning model trained to predict a gene interaction profile for a range of compounds, and
building the second fingerprint using the genes returned by the search and the predicted genes; and
filtering a group comprising the first candidate compounds and the second candidate compounds using the first fingerprints and the second fingerprints to identify the first optimum compound and to identify a second optimum compound for targeting the one or more second target genes; wherein filtering the group of first candidate compounds and second candidate compounds comprises comparing each of the first and second fingerprints to an ideal fingerprint of a theoretical tool compound, where each fingerprint comprises a polypharmacology fingerprint that describes which genes the compound is associated with.

9. A system for identifying a compound for targeting one or more target genes, the system comprising:
a compound search module configured to search a database for first candidate compounds that each target one or more first target genes;
a fingerprint module configured to generate a first fingerprint for each first candidate compound, the fingerprint module comprising:
a gene search module configured to search the database for genes associated with the first candidate compound, and
a prediction module configured to predict genes associated with the first candidate compound using a machine learning model trained to predict a gene interaction profile for a range of compounds, fingerprint module configured to build the first fingerprint using the genes returned by the gene search module and the prediction module; and
a filter module configured to filter the first candidate compounds using the first fingerprints to identify a first optimum compound for targeting the one or more first target genes, wherein filtering the first candidate compounds comprises:
combining filtering by comparing each of the first fingerprints to an ideal fingerprint of a theoretical tool compound and filtering by using metrics generated from the first fingerprints,
wherein the metrics comprise one or more of: molar weight (MW), the logarithm of the partition coefficient (logP), the number of hydrogen bond acceptors or donors, enzyme activity such as values of the half maximal inhibitory concentration (IC50) of the compound or the half maximal effective concentration of the compound (EC50) in assay, selectivity of a compound for a target gene, number of unwanted genes that the compound affects, potency of the compound for a gene, solubility, and cell data providing an indication of the activity of a compound in a cellular assay, and
where each fingerprint comprises a polypharmacology fingerprint that describes which genes the compound is associated with.

10. A computer-readable medium storing code that, when executed by a computer, causes the computer to perform the method of any one of claims 1-8

## Patentansprüche

1. Computerimplementiertes Verfahren zur Identifizierung einer Verbindung zum Abzielen auf ein oder mehrere Zielgene, wobei das Verfahren Folgendes umfasst:
Durchsuchen einer Datenbank nach ersten Kandidatenverbindungen, die jeweils auf ein oder mehrere erste Zielgene abzielen;
Erzeugen eines ersten Fingerabdrucks für jede erste Kandidatenverbindung durch:
Durchsuchen der Datenbank nach Genen, die mit der ersten Kandidatenverbindung in Verbindung stehen,
Vorhersagen von Genen, die mit der ersten Kandidatenverbindung in Verbindung stehen, unter Verwendung eines Modells für maschinelles Lernen, das zur Vorhersage eines Gen-Wechselwirkungs-Profils für einen Bereich von Verbindungen trainiert ist, und
Erstellen des ersten Fingerabdrucks unter Verwendung der Gene, die durch die Suche gefunden werden, und der vorhergesagten Gene; und
Filtern der ersten Kandidatenverbindungen unter Verwendung der ersten Fingerabdrücke, um eine erste optimale Verbindung zum Abzielen auf das eine oder die mehreren ersten Zielgene zu identifizieren, wobei das Filtern der ersten Kandidatenverbindungen Folgendes umfasst:
Kombinieren des Filterns durch Vergleichen von jedem der ersten Fingerabdrücke mit einem idealen Fingerabdruck einer Verbindung eines theoretischen Werkzeugs und des Filterns durch Verwendung von Messdaten, die aus den ersten Fingerabdrücken erzeugt werden,
wobei die Messdaten eines oder mehrere von Folgenden umfassen:
Molekulargewicht (MW), den Logarithmus des Verteilungskoeffizienten (logP), die Anzahl von Wasserstoffbrücken-Akzeptoren oder -Donoren, Enzymaktivität wie zum Beispiel Werte der halbmaximalen inhibitorischen Konzentration (IC50) der Verbindung oder der halbmaximalen effektiven Konzentration der Verbindung (EC50) im Assay, Selektivität einer Verbindung für ein Zielgen, Anzahl von unerwünschten Genen, die die Verbindung beeinflusst, Wirksamkeit der Verbindung für ein Gen,
Löslichkeit und Zelldaten, die ein Anzeichen der Aktivität einer Verbindung in einem zellulären Assay bereitstellen,
wo jeder Fingerabdruck einen polypharmakologischen Fingerabdruck umfasst, der beschreibt, mit welchen Genen die Verbindung in Verbindung steht.

2. Computerimplementiertes Verfahren nach Anspruch 1, wobei jeder Fingerabdruck für eine Verbindung das Ausmaß der Hochregulierung von jedem einer Anzahl von Genen, die mit der Verbindung in Verbindung stehen, darstellt.

3. Computerimplementiertes Verfahren nach einem vorstehenden Anspruch, wobei jeder Fingerabdruck für eine Verbindung das Ausmaß darstellt, in dem jedes einer Anzahl von Genen durch die Verbindung inhibiert wird.

4. Computerimplementiertes Verfahren nach einem vorstehenden Anspruch, wobei das Modell ein neuronales Netz umfasst.

5. Computerimplementiertes Verfahren nach einem vorstehenden Anspruch, wobei das Vergleichen das Berechnen eines Ähnlichkeits-Scores umfasst.

6. Computerimplementiertes Verfahren nach einem vorstehenden Anspruch, das das Identifizieren einer ersten Kandidatenverbindung, die mit der Verbindung des theoretischen Werkzeugs am ähnlichsten ist, als die erste optimale Verbindung umfasst.

7. Computerimplementiertes Verfahren nach einem vorstehenden Anspruch, wobei das Erzeugen der ersten Fingerabdrücke das Erhalten von Metadaten über eine oder mehrere der ersten Kandidatenverbindungen umfasst, wobei die Metadaten Daten der klinischen Testphase, einen Arzneimittelnamen oder eine Arzneimitteleigenschaft oder Informationen von einem Verbindungsanbieter umfassen.

8. Computerimplementiertes Verfahren nach einem vorstehenden Anspruch, das Folgendes umfasst:
Durchsuchen der Datenbank nach zweiten Kandidatenverbindungen, die jeweils auf ein oder mehrere zweite Zielgene abzielen;
Erzeugen eines zweiten Fingerabdrucks für jede zweite Kandidatenverbindung durch:
Durchsuchen der Datenbank nach Genen, die mit der zweiten Kandidatenverbindung in Verbindung stehen, und
Vorhersagen von Genen, die mit der zweiten Kandidatenverbindung in Verbindung stehen, unter Verwendung eines Modells für maschinelles Lernen, das zur Vorhersage eines Gen-Wechselwirkungs-Profils für einen Bereich von Verbindungen trainiert ist, und
Erstellen des zweiten Fingerabdrucks unter Verwendung der Gene, die durch die Suche gefunden werden, und der vorhergesagten Gene; und
Filtern einer Gruppe, die die ersten Kandidatenverbindungen und die zweiten Kandidatenverbindungen umfasst, unter Verwendung der ersten Fingerabdrücke und der zweiten Fingerabdrücke, um die erste optimale Verbindung zu identifizieren und eine zweite optimale Verbindung zum Abzielen auf das eine oder die mehreren zweiten Zielgene zu identifizieren, wobei das Filtern der Gruppe der ersten Kandidatenverbindungen und zweiten Kandidatenverbindungen das Vergleichen von jedem der ersten und zweiten Fingerabdrücke mit einem idealen Fingerabdruck einer Verbindung eines theoretischen Werkzeugs umfasst, wo jeder Fingerabdruck einen polypharmakologischen Fingerabdruck umfasst, der beschreibt, mit welchen Genen die Verbindung in Verbindung steht.

9. System zur Identifizierung einer Verbindung zum Abzielen auf ein oder mehrere Zielgene, wobei das System Folgendes umfasst:
ein Modul der Verbindungssuche, das zur Durchsuchung einer Datenbank nach ersten Kandidatenverbindungen konfiguriert ist, die jeweils auf ein oder mehrere erste Zielgene abzielen;
ein Fingerabdruckmodul, das zur Erzeugung eines ersten Fingerabdrucks für jede erste Kandidatenverbindung konfiguriert ist, wobei das Fingerabdruckmodul Folgendes umfasst:
ein Modul zur Gensuche, das zur Durchsuchung der Datenbank nach Genen konfiguriert ist, die mit der ersten Kandidatenverbindung in Verbindung stehen, und
ein Vorhersagemodul, das zur Vorhersage von Genen, die mit der ersten Kandidatenverbindung in Verbindung stehen, unter Verwendung eines Modells für maschinelles Lernen konfiguriert ist, das zur Vorhersage eines Gen-Wechselwirkungs-Profils für einen Bereich von Verbindungen trainiert ist, wobei das Fingerabdruckmodul zum Erstellen des ersten Fingerabdrucks unter Verwendung der Gene, die durch das Modul zur Gensuche und das Vorhersagemodul gefunden werden, konfiguriert ist; und
ein Filtermodul, das zum Filtern der ersten Kandidatenverbindungen unter Verwendung der ersten Fingerabdrücke konfiguriert ist, um eine erste optimale Verbindung zum Abzielen auf das eine oder die mehreren ersten Zielgene zu identifizieren, wobei das Filtern der ersten Kandidatenverbindungen Folgendes umfasst:
Kombinieren des Filterns durch Vergleichen von jedem der ersten Fingerabdrücke mit einem idealen Fingerabdruck einer Verbindung eines theoretischen Werkzeugs und des Filterns durch Verwendung von Messdaten, die aus den ersten Fingerabdrücken erzeugt werden,
wobei die Messdaten eines oder mehrere von Folgenden umfassen:
Molekulargewicht (MW), den Logarithmus des Verteilungskoeffizienten (logP), die Anzahl von Wasserstoffbrücken-Akzeptoren oder -Donoren, Enzymaktivität wie zum Beispiel Werte der halbmaximalen inhibitorischen Konzentration (IC50) der Verbindung oder der halbmaximalen effektiven Konzentration der Verbindung (EC50) im Assay, Selektivität einer Verbindung für ein Zielgen, Anzahl von unerwünschten Genen, die die Verbindung beeinflusst, Wirksamkeit der Verbindung für ein Gen,
Löslichkeit und Zelldaten, die ein Anzeichen der Aktivität einer Verbindung in einem zellulären Assay bereitstellen, und
wo jeder Fingerabdruck einen polypharmakologischen Fingerabdruck umfasst, der beschreibt, mit welchen Genen die Verbindung in Verbindung steht.

10. Computerlesbares Medium zum Speichern von Code, der bei Ausführung durch einen Computer bewirkt, dass der Computer das Verfahren nach einem der Ansprüche 1-8 ausführt.

## Revendications

1. Procédé d'identification d'un composé pour cibler un ou plusieurs gènes cibles, mis en oeuvre par l'informatique, le procédé comprenant :
rechercher dans une base de données des premiers composés candidats qui ciblent chacun un ou plusieurs gènes cibles ;
générer une première empreinte génétique pour chaque premier composé candidat en :
recherchant dans la base de données des gènes associés au premier composé candidat,
prédire des gènes associés au premier composé candidat en utilisant un modèle d'apprentissage machine formé pour prédire un profil d'interaction de gènes pour une gamme de composés, et
constituer la première empreinte génétique en utilisant les gènes renvoyés par la recherche et les gènes prédits ; et
filtrer les premiers composés candidats en utilisant les premières empreintes génétiques afin d'identifier un premier composé optimal pour cibler l'un ou plusieurs premiers gènes cibles, dans lequel filtrer les premiers composés candidats comprend :
combiner le filtrage en comparant chacune des premières empreintes génétiques à une empreinte génétique idéale d'un composé d'outil théorique et filtrer en utilisant une métrique générée à partir des premières empreintes génétiques,
dans lequel la métrique comprend un ou plusieurs d'entre : une masse moléculaire (MW), le logarithme de coefficient de partage (logP), le nombre d'accepteurs ou donneurs de liaison hydrogène, l'activité enzymatique telle que des valeurs de la moitié de la concentration inhibitrice maximale (CI50) du composé ou de la moitié de la concentration efficace maximale (CE50) du composé dans un essai, la sélectivité d'un composé pour un gène cible, le nombre de gènes indésirables que le composé affecte, la puissance du composé pour un gène, la solubilité, et des données cellulaires donnant une indication de l'activité du composé dans un essai cellulaire,
dans lequel chaque empreinte génétique comprend une empreinte génétique de polypharmacologie qui décrit avec quels gènes le composé est associé.

2. Procédé mis en oeuvre par l'informatique selon la revendication 1, dans lequel chaque empreinte génétique pour un composé représente l'étendue de la régulation à la hausse de chacun d'un nombre de gènes associés au composé.

3. Procédé mis en oeuvre par l'informatique selon l'une quelconque des revendications précédentes, dans lequel chaque empreinte génétique pour un composé représente l'étendue selon laquelle chacun d'un nombre de gènes sont inhibés par le composé.

4. Procédé mis en oeuvre par l'informatique selon l'une quelconque des revendications précédentes, dans lequel le modèle comprend un réseau neuronal.

5. Procédé mis en oeuvre par l'informatique selon l'une quelconque des revendications précédentes, dans lequel le fait de comparer comprend calculer un score de similarité.

6. Procédé mis en oeuvre par l'informatique selon l'une quelconque des revendications précédentes, comprenant identifier un premier composé candidat qui ressemble le plus au composé d'outil théorique comme le premier composé optimal.

7. Procédé mis en oeuvre par l'informatique selon l'une quelconque des revendications précédentes, dans lequel générer les premières empreintes génétiques comprend obtenir des métadonnées au sujet d'un ou de plusieurs premiers composés candidats, dans lequel les métadonnées comprennent des données de phase d'essai clinique, un nom ou une propriété de médicament ou des informations d'un vendeur de composés.

8. Procédé mis en oeuvre par l'informatique selon l'une quelconque des revendications précédentes, comprenant :
rechercher dans la base de données des deuxièmes composés candidats qui ciblent chacun un ou plusieurs deuxièmes gènes cibles ;
générer une deuxième empreinte génétique pour chaque deuxième composé candidat en :
recherchant dans la base de données des gènes associés au deuxième composé candidat, et
prédisant des gènes associés au deuxième composé candidat en utilisant un modèle d'apprentissage machine formé pour prédire un profil d'interaction de gènes pour une gamme de composés, et
constituer la deuxième empreinte génétique en utilisant les gènes renvoyés par la recherche et les gènes prédits ; et
filtrer un groupe comprenant les premiers composés candidats et les deuxièmes composés candidats en utilisant les premières empreintes génétiques et les deuxièmes empreintes génétiques pour identifier le premier composé optimal et pour identifier un deuxième composé optimal pour cibler l'un ou plusieurs deuxièmes gènes cibles ; dans lequel filtrer le groupe de premiers composés candidats et de deuxièmes composés candidats comprend comparer chacune des premières et des deuxièmes empreintes génétiques à une empreinte génétique idéale d'un composé d'outil théorique, où chaque empreinte génétique comprend une empreinte génétique de polypharmacologie qui décrit avec quels gènes le composé est associé.

9. Système d'identification d'un composé pour cibler un ou plusieurs gènes cibles, le système comprenant :
un module de recherche de composés configuré pour rechercher dans une base de données des premiers composés candidats qui ciblent chacun un ou plusieurs premiers gènes cibles ;
un module d'empreinte génétique configuré pour générer une première empreinte génétique pour chaque premier composé candidat, le module d'empreinte génétique comprenant :
un module de recherche de gènes configuré pour rechercher dans la base de données des gènes associés au premier composé candidat, et
un module de prédiction configuré pour prédire des gènes associés au premier composé candidat en utilisant un modèle d'apprentissage machine formé pour prédire un profil d'interaction de gènes pour une gamme de composés, le module d'empreinte génétique étant configuré pour constituer la première empreinte génétique en utilisant les gènes renvoyés par le module de recherche de gènes et le module de prédiction ; et
un module de filtre configuré pour filtrer les premiers composés candidats en utilisant les premières empreintes génétiques pour identifier un premier composé optimal pour cibler l'un ou plusieurs premiers gènes cibles, dans lequel filtrer les premiers composés candidats comprend :
combiner le filtrage en comparant chacune des premières empreintes génétiques à une empreinte génétique idéale d'un composé d'outil théorique et filtrer en utilisant une métrique générée à partir des premières empreintes génétiques,
dans lequel la métrique comprend un ou plusieurs d'entre : une masse moléculaire (MW), le logarithme de coefficient de partage (logP), le nombre d'accepteurs ou donneurs de liaison hydrogène, l'activité enzymatique telle que des valeurs de la moitié de la concentration inhibitrice maximale (CI50) du composé ou de la moitié de la concentration efficace maximale (CE50) du composé dans un essai, la sélectivité d'un composé pour un gène cible, le nombre de gènes indésirables que le composé affecte, la puissance du composé pour un gène, la solubilité, et des données cellulaires donnant une indication de l'activité du composé dans un essai cellulaire, et
dans lequel chaque empreinte génétique comprend une empreinte génétique de polypharmacologie qui décrit avec quels gènes le composé est associé.

10. Support lisible par ordinateur stockant du code qui, lorsque exécuté par un ordinateur, fait que l'ordinateur met en oeuvre le procédé selon l'une quelconque des revendications 1-8.
